# EUROPEAN PATENT SPECIFICATION

(11) **EP 3 603 487 B1**
(45) Date of publication and mention of the grant of the patent: **16.03.2022**
(21) Application number: 19189563.0
(22) Date of filing: 01.08.2019
(51) Int. Cl.: A61B 3/12, A61B 3/00, A61B 3/15

(54) **FUNDUS IMAGING APPARATUS AND METHOD OF PRODUCING COMPOSITE IMAGE COMPOSED OF FUNDUS IMAGES**
FUNDUSBILDGEBUNGSVORRICHTUNG UND VERFAHREN ZUR HERSTELLUNG EINES ZUSAMMENGESETZTEN BILDES AUS FUNDUSBILDERN
APPAREIL D'IMAGERIE DE FOND DE L' IL ET PROCÉDÉ DE PRODUCTION D'UNE IMAGE COMPOSITE CONSTITUÉE D'IMAGES DE FOND DE L' IL

(30) Priority: 03.08.2018 JP 2018147142
(43) Date of publication of application: 05.02.2020
(73) Proprietor: Nidek Co., Ltd., Gamagori-shi, Aichi, 443-0038 (JP)
(72) Inventor: MURASE, Yuji, Aichi (JP); FUJIU, Kenshiro, Aichi (JP)
(74) Representative: Hoefer & Partner Patentanwälte mbB

(56) References cited:
- EP-A2- 3 300 654
- WO-A1-2016/009739
- WO-A2-2015/087332
- JP-A- 2018 051 244
- JP-B2- 4 437 664

## Description

### TECHNICAL FIELD

The present disclosure relates to a fundus imaging apparatus and a method of producing a composite image composed of a plurality of fundus images.

### BACKGROUND

Various apparatuses such as a fundus camera, a scanning laser ophthalmoscope (SLO), and an optical coherence tomography (OCT) are known as fundus imaging apparatuses. In addition, in the ophthalmology field, a method of improving an image quality compared to the original fundus image by producing a composite image in which a plurality of fundus images continuously captured by these apparatuses are added, is known (for example, refer to JP-A-2018-51244).

For example, since a state of a subject eye such as a pupil state changes while capturing the fundus image continuously, it is conceivable that an image which is not preferable to be composed with another image is mixed in at least a part of a plurality of fundus images obtained by continuous imaging.

From EP 3 300 654 A2, a fundus image observation program is known in which a computer acquires in advance at least one first fluorescent angiographic image photographed on the basis of a first photographing trigger signal, and at least one second fluorescent angiographic image photographed on the basis of a second photographing trigger signal whose output timing is different from the first photographing trigger signal, as one set of image group data in a memory in a fluorescent imaging procedure for an examinee's eye. A fluorescent angiographic image photographed at any angiography timing is selectively displayed on a monitor from the first and second fluorescent angiographic images included in the image group data on the basis of the image group data.

From JP 2018 051244 A, a fundus image observation program is known in which a computer acquires at least one first fluorescent contrast image photographed based on a first photographing trigger signal in a series of fluorescent imaging photography of an eye to be examined and at least one second fluorescent contrast image photographed based on a second photographing trigger signal of output timing different from the first photographing trigger signal in a memory in advance as one image group data. The fluorescent contrast image photographed at an arbitrary imaging period out of the first fluorescent contrast image and the second fluorescent contrast image included in the image group data is selectively displayed on a monitor based on the image group data.

From WO 2016/009739 A1, a visual function testing device is known which comprises an illuminating optical system, a biological information detector, and an evaluation information generation unit. The illuminating optical system comprises a light scanner that is disposed in the path of laser light that is output from a laser light source, and the retina of the subject's eye is illuminated with the laser light that passes through the optical scanner. The biological information detector detects biological information that represents the response of the subject to the illumination with the laser light. The evaluation information generation unit generates evaluation information that relates to the visual function of the subject's eye on the basis of the generated biological information.

### SUMMARY

An object of the present disclosure is to provide a fundus imaging apparatus that can produce a good composite image composed of a plurality of fundus images and a method of producing the composite image.

The solution of this object is achieved by the features of the independent claims. The dependent claims contain advantageous embodiments of the present invention.

According to the present disclosure, it is possible to produce a good composite image composed of a plurality of fundus images.

### BRIEF DESCRIPTION OF DRAWINGS

Fig. 1 is a diagram illustrating an appearance of a fundus imaging apparatus according to an application example.
Fig. 2 is a diagram illustrating an imaging optical system according to the application example.
Fig. 3 is a diagram illustrating the imaging optical system in a state in which a wide angle attachment is attached to.
Fig. 4 is a diagram illustrating a control system of an SLO according to the application example.
Fig. 5 is a view illustrating an example of a fundus image.
Fig. 6 is a view illustrating an example of a fundus image in which an influence of vignetting occurs in the peripheral portion by a miosis.
Fig. 7 is a flowchart illustrating an operation performed by the apparatus according to the application example.
Fig. 8 is a flowchart illustrating continuous imaging processing according to the application example.
Fig. 9 is a flowchart illustrating pause processing according to the application example.
Fig. 10 is a diagram for explaining an example of a method of performing pre-notice for starting or restarting the imaging using a fixation target.
Figs. 11A to 11C are diagrams for describing modification examples.

### DETAILED DESCRIPTION

### (SUMMARY)

Embodiments of the present disclosure will be described with reference to the drawings. First, an embodiment of a fundus imaging apparatus will be described.

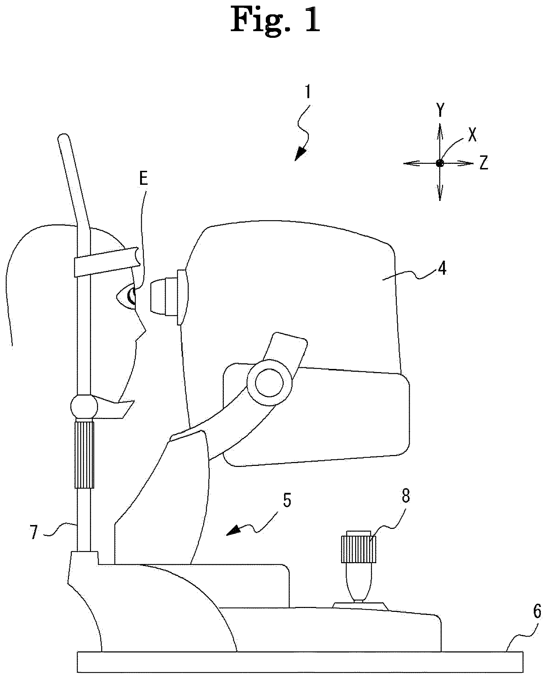

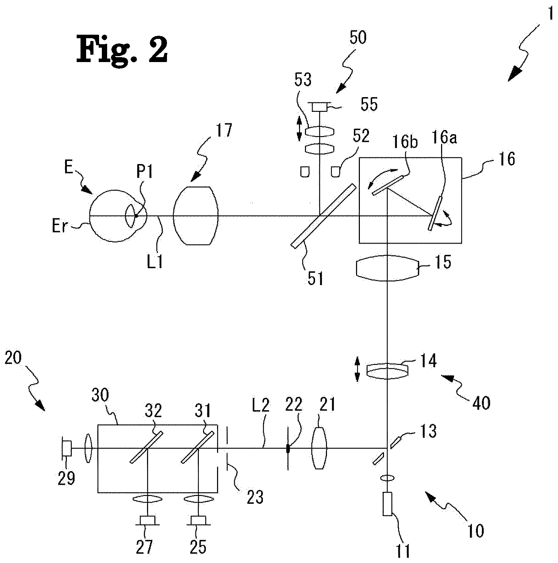

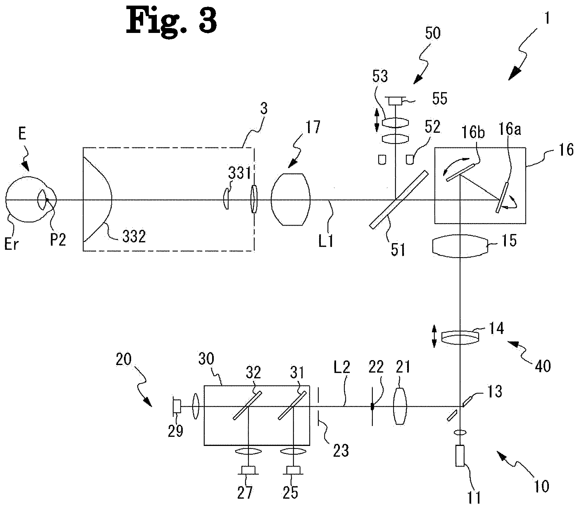

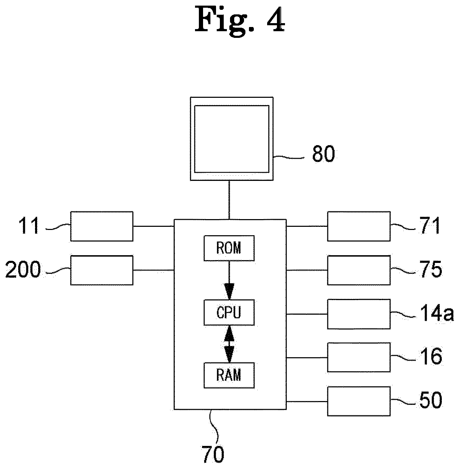

The fundus imaging apparatus (refer to Fig. 1) according to the present embodiment is used for imaging a fundus of a subject eye. The fundus imaging apparatus includes at least an imaging optical system (for example, refer to Fig. 2 and Fig. 3), a control unit (also referred to as a "processor", for example, refer to Fig. 4), and an image processing unit (an image processing processor). The control unit may be used as the image processing unit. Additionally, the fundus imaging apparatus may include at least one of an observation optical system, a fixation optical system, an output unit, and a drive unit.

### <Imaging Optical System>

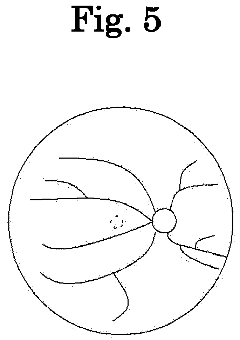

The imaging optical system irradiates the fundus of the subject eye with illumination light. The imaging optical system further captures a fundus image (refer to Fig. 5) based on return light of the illumination light from the fundus.

The imaging optical system may capture a front image of the fundus as the fundus image. The imaging optical system may include a front imaging optical system (refer to Figs. 2 and 3) that captures the front image of the fundus. When capturing the front image, visible light may be used as the illumination light, and infrared light may be used as the illumination light. In addition, not necessarily being limited thereto, the imaging optical system may include an OCT optical system (not illustrated) that acquires OCT data of the fundus based on a spectrum interference signal of the return light and reference light. Alternatively, the fundus imaging apparatus may include two imaging optical systems of a front imaging optical system and an OCT optical system. Two imaging optical systems may be capable of imaging the subject eye at the same time.

The imaging optical system may include an emission optical system and a light receiving optical system (refer to Fig. 2 and Fig. 3). The emission optical system irradiates the fundus of the subject eye with the illumination light. Additionally, the emission optical system may include a light source (illumination light source) that emits the illumination light. The light receiving optical system may include a light receiving element that receives fundus reflection light of the illumination light. In this case, a signal from the light receiving element is input to the image processing unit, and as a result, a fundus image of the subject eye is acquired (produced).

If the imaging optical system is the front imaging optical system, the imaging optical system may be a scanning optical system that performs the imaging by scanning the fundus with the illumination light. In addition, the imaging optical system may be a non-scanning optical system. Example of the scanning optical system include a spot scan type optical system and a line scan type optical system. In the spot scan type optical system, the fundus is two-dimensionally scanned by the spot illumination light. In the line scan type optical system, the scanning is performed by line-shape illumination light. The fundus may be linearly scanned by the line-shape illumination light or the fundus may be rotationally scanned. In the case of rotational scanning, the center of rotation may be an optical axis of the imaging optical system. In scanning optical system, any one of a point light receiving element, a line sensor, a two-dimensional light receiving element (imaging element) and the like can be appropriately adopted as the light receiving element. In addition, an example of the non-scanning optical system include a general optical system of a fundus camera.

If the imaging optical system is the OCT optical system, the OCT data is acquired based on the light interference between the light radiated to the fundus and the reference light. The OCT optical system may be an FD-OCT. An SD-OCT, an SS-OCT, and the like are known as the FD-OCT. Hereinafter, the description will be made using the SD-OCT unless otherwise noted. However, not necessarily limited thereto, and each type of the OCT optical system can be adopted as a part or all of the imaging optical system.

### <Observation Optical System>

The fundus imaging apparatus may include the observation optical system. An anterior portion may be observed or the fundus may be observed by the observation optical system. The observation optical system irradiates the subject eye with invisible light (for example, infrared light) as the observation light, and observes the subject eye based on the reflection light of the observation light (obtains an observation image). The image (the observation image) obtained by observation through the observation optical system may be an image of the anterior portion or a front image of the fundus observed from a predetermined direction. In the present embodiment, the observation optical system may be partially or entirely shared with the imaging optical system. In addition, the observation optical system may be an optical system completely independent of the imaging optical system. The fundus imaging apparatus may include both the anterior portion observation optical system and the fundus observation optical system as the observation optical system. Various imaging conditions may be adjusted using the observation image obtained via the observation optical system. One of the various imaging conditions may include an alignment state or a focus state.

### <Fixation Optical System>

In addition, the fundus imaging apparatus may include a fixation optical system. The fixation optical system may be an internal fixation optical system presenting an internal fixation target, or may be an external fixation optical system (external fixation lamp) presenting an external fixation target. The fixation optical system which projects the internal fixation target onto the subject eye shares some optical paths with the imaging optical system. The fixation optical system includes at least a light source for forming a fixation target. The fixation position by the fixation target may be changeable. The fixation optical system may include any one of a projector or a display to form the fixation target. In addition, the imaging optical system may be used as the fixation optical system (details will be described later in the application example).

### <Control Unit>

The control unit is a processing device (a processor) that performs control processing and arithmetic processing for each unit in the fundus imaging apparatus. For example, the control unit is realized by a central processing unit (CPU), a memory, and the like.

### <Image Processing Unit>

The image processing unit performs at least one of the production of a fundus image and various image processing for the fundus image. In the present embodiment, the control unit may act as an image processing unit. Of course, the image processing unit may be separate from the control unit.

### <Imaging Operation>

The control unit controls the imaging optical system, and continuously captures a plurality of fundus images based on an imaging trigger. At this time, the fundus image may be repeatedly captured at a constant period (a frame rate). The continuous imaging may be continued until a predetermined time has elapsed from the start of imaging, may be continued until a predetermined number of fundus images are obtained, or may be continued until a predetermined operation is input.

In the present embodiment, the imaging trigger may be a releasing operation by the examiner. Further, without necessarily requiring an operation of the examiner, a fact that predetermined conditions such as completion of alignment, completion of focus adjustment, and the like are satisfied may be used as the imaging trigger.

In the fundus imaging apparatus, the continuous imaging may be one of a plurality of imaging methods prepared in advance. Besides the continuous imaging, other imaging methods such as one-shot imaging may be prepared. In the one-shot imaging, the illumination light may be intermittently radiated to capture one frame of a fundus image.

### <Production of Composite Image>

A composite image of a plurality of fundus images acquired by the continuous imaging is produced by an image processing unit. The composite image may be, for example, an addition image or a super resolution image. The addition image is not limited to the addition image by simple addition processing, and may be an addition average image produced by addition averaging processing. The addition average image may be a weighted average image.

A position deviation between the plurality of fundus images that are the basis of the composite image may be appropriately corrected when producing the composite image. For example, the position deviation may be corrected using a known matching processing such as a phase limited correlation.

In the present embodiment, every time a sheet of new fundus image is captured by the continuous imaging, a composite image based on a plurality of sheets of fundus images captured so far may be produced by the image processing unit. In this case, a new composite image may be displayed on the monitor every time the composite image is updated in the continuous imaging. In this way, the examiner can easily grasp the quality of the imaging during the continuous imaging. Of course, not limited thereto, the composite image may be produced after the continuous imaging is completed and a plurality of fundus images are stored in the memory.

Here, in some cases, if a plurality of fundus images that are the basis of the composite image include an image with poor image quality compared to other images or an image with vignetting on the peripheral portions of the image, it may be difficult to obtain a good composite image. Whether the image with poor image quality or the image with vignetting on the peripheral portions of the image is captured or not may be caused by the state of the subject eye during imaging.

### <Stop Determination of Continuous Imaging based on Evaluation Information on State of Subject Eye>

Therefore, in the present embodiment, during the continuous imaging, the control unit sequentially acquires information (referred to as "evaluation information") that is used an evaluation index of the state of the subject eye. At the same time, the control unit determines whether or not to stop the continuous imaging based on the evaluation information. According to the result of determination, the continuous imaging may be continued or stopped. When the continuous imaging is stopped, the continuous imaging may be restarted after a certain time elapsed, or may be ended at that time. In the present embodiment, since the imaging is stopped if the state of the subject eye is not good, the burden of unnecessary imaging given to an examinee is suppressed. In addition, since an image which is not preferable to be composed with another image is suppressed to be mixed in a plurality of images which are the basis of composite image, it becomes easy to obtain a good composite image.

In addition, if the continuous imaging is ended at that time according to the result of determination, the number of fundus images is different for each continuous imaging. Therefore, if the addition image is produced by the simple addition, it is preferable to perform normalization processing in order to suppress the variation in the luminance caused by the number of fundus images.

The evaluation information in the present embodiment is information relating to the state of the subject eye that changes during the continuous imaging and affects the fundus image. As a specific example, the evaluation information may be information relating to at least one of a pupil state, an alignment state, and a fixation state in the subject eye. The evaluation information may be acquired based on the fundus image or the anterior portion image obtained during the continuous imaging.

In addition, the control unit may determine whether or not to stop the continuous imaging based on one evaluation information acquired at a certain timing, or may determine based on a plurality of evaluation information acquired within the time required for capturing a plurality of fundus images. For example, an average value of a plurality of evaluation information acquired within a time required to capture a plurality of fundus images may be used for the determination. In the former, since the stop determination is performed based on the state of the subject eye in a shorter-term, it becomes difficult that the image which is not preferable to be composed with another image is mixed in. On the other hand, in the latter, since the stop determination is performed based on the state of the subject eye in a longer-term, the efficiency of imaging hardly decreases.

### <Specific example of Evaluation Information>

### (1) Evaluation Information relating to Pupil State

In the above-described specific example, it is preferable that the evaluation information relating to the pupil state be obtained when the visible light is used as the illumination light (imaging light). When the visible light is used as the illumination light, since the miosis occurs when the continuous imaging is started, the pupil state changes during the continuous imaging.

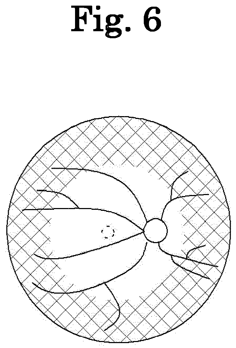

As the miosis occurs more, it becomes difficult for the illumination light to reach the peripheral portion of the shooting range, and thus, it is difficult to obtain the return light. Thus, for example, the intensity of return light decreases from the peripheral portion side. As the miosis progresses, the peripheral portion of the fundus image may be affected by the vignetting (refer to Fig. 6). Therefore, the information on the luminance of the fundus image may be used as the evaluation information relating to the miosis. The information on the luminance may be a histogram-based value relating to the luminance (for example, any one of an average value, a median value, a most-frequent value, a variance, a deviation, and the like). In addition, the information on the luminance does not necessarily need to be obtained from the entire fundus image, but may be obtained from a part of the image (for example, the image peripheral portion).

In addition, when the anterior portion is observed during the continuous imaging, the evaluation information relating to the miosis may be acquired from the anterior portion observation image. For example, pupil diameter information detected from the anterior portion observation image may be used as the evaluation information.

### (2) Evaluation Information relating to Alignment State

In addition, the burden is accumulated to the examinee as the time of continuous imaging elapses. Therefore, it is considered that the stability of fixation and the stability of alignment state decrease with the lapse of time of continuous imaging.

It is considered that, if the alignment state changes, the luminance of each part in the fundus image is changed. In detail, when an exit pupil of the apparatus and the pupil of the subject eye deviate from each other, the peripheral portion of the fundus image may be affected by vignetting or the like. Therefore, the information on the luminance of the fundus image may be used as the evaluation information relating to the alignment state. In addition, it is considered that a position of the tissue of the anterior portion in the anterior portion observation image is changed depending on the alignment state. Therefore, the information on the position deviation of the tissue due to the continuous imaging may be used as the evaluation information relating to alignment state. For example, the position deviation may be a position deviation from the start of continuous imaging. At this time, for example, a characteristic portion such as a pupil center may be detected from the anterior portion observation image, and the information on the position of the characteristic portion may be used as the evaluation information. In addition, information on the position of the alignment index image formed by projecting the alignment index from the index projection optical system may be used as the evaluation information relating to the alignment state. Various indices such as the index formed on the corneal apex or the index formed around the corneal apex are known as the alignment index, and those can be applied to the present embodiment as appropriate. In this case, the fundus imaging apparatus in the present embodiment may include an index projection optical system that projects the alignment index onto the subject eye.

If each of the evaluation information relating to the pupil state and the evaluation information relating to the alignment state is obtained from the fundus image only, it is difficult to acquire the two in a completely distinguished manner. However, as long as the image which is not preferable to be composed with another image can be specified, the evaluation information relating to the pupil state and the evaluation information relating to the alignment state do not necessarily need to be strictly distinguished.

### (3) Evaluation Information relating to Fixation State

It is considered that the position of the tissue in the fundus image is changed when the fixation state changes. Therefore, the information on the position deviation in the fundus image which is the information on the position deviation of the tissue due to the continuous imaging may be used as the evaluation information relating to the fixation state. The position deviation may be the position deviation from the start of the continuous imaging. For example, the information on the position deviation of the characteristic portions such as macula, papilla, blood vessels, or the like may be acquired, or the entire information on the position deviation may be acquired.

Examples of evaluation information include the information on the luminance and information on the position deviation, but not necessarily be limited thereto, and information indicating the temporal change of those information may be used as the evaluation information. For example, a time-based differential value of each information and a cumulative value of the differential values may be used as the evaluation information.

The evaluation information obtained as described above may be compared with a threshold value, for example, by the control unit. Then, whether or not to stop the continuous imaging is determined according to the result of comparison. The threshold value may be appropriately set based on an experiment or the like.

### <Restart of Continuous Imaging>

The control unit may restart the continuous imaging after stopping the continuous imaging based on the determination made during the continuous imaging. When the continuous imaging is performed using the visible light, a paused period until the restart is effective for miosis recovery. In addition, since fatigue due to the continuous imaging is recovered, the paused period is also effective in the stabilization of the alignment state and the fixation state. Therefore, after the restart, it is easy to capture the fundus image under the good state of the subject eye (the pupil state, the alignment state, and the fixation state).

The restart may be based on, for example, an operation by the examiner. In addition, the control unit may sequentially acquire the evaluation information relating to the state of the subject eye even while the continuous imaging is stopped and may determine whether or not to restart the continuous imaging based on the evaluation information acquired during the stop of the continuous imaging. For example, when the evaluation information acquired during the stop of the continuous imaging becomes equal to that at the start of the continuous imaging, the determination to restart may be performed. As a result of determination to restart, the imaging may be may be restarted automatically. Not being necessarily limited to the above-described, as the result of determination to restart, an operation for restarting may be guided to the examiner.

If the continuous imaging is restarted, the imaging may be performed again by taking over the imaging result before the stop (the fundus image captured before the stop, the result of the real time addition, and the like).

### <Operation during Stop>

With continuous imaging being stopped, the control unit may stop the emission of illumination light (the imaging light). If the imaging light is visible light, the stop of emission is useful for restoring the miosis. The observation of the subject eye via the observation optical system may be continued.

When restarting the continuous imaging after stopping temporarily, the control unit may present a fixation target during stop of the continuous imaging. However, the fixation target does not need to be presented at all times during the stop, and may be presented at least some time before restarting and just before restarting.

### <Pre-notice for Starting or Restarting Continuous Imaging>

When starting or restarting the continuous imaging, the control unit may perform a pre-notice for starting or restarting the continuous imaging via the output unit. Since the continuous imaging takes a relatively long time for imaging, it is considered that the burden to the examinee is relatively large. Therefore, by performing the pre-notice before imaging, for example, it is possible to prompt the examiner to maintain the open eye and fixation.

The output unit outputs the information to any one of the examiner or the examinee. Typical examples of the output unit include a monitor and a speaker. In addition, the fixation optical system may be used as the output unit (details will be described later).

In order to suppress an error due to a blink of the subject eye, the pre-notice may include information prompting the subject eye to blink. For example, as a pre-notice by a voice, a message such as "Imaging is performed. Please blink and open the eye widely." may be output from the speaker.

### <Detection of Blink >

In addition, after the pre-notice is performed, the control unit may further detect a blink, and may start or restart the continuous imaging after detecting the blink. The blink may be detected based on the observation image of the anterior portion or the fundus. If the continuous imaging is started or restarted after the blinks are detected, the imaging errors due to the blink is reduced. However, it is preferable that the start or restart of the imaging is performed at a time approximately 0.5 seconds after the blink rather than immediately after the detection of the blink. Since the fixation is difficult to stabilize immediately after the blink, the subject eye moves at the time of imaging, and thus, the noise (flare or the like) easily occurs in the captured image. On the contrary, as described above, the occurrence of noise is suppressed by performing the imaging with a short time after the detection of blink not immediately after the blink. The time from the timing of the detection of blink to the timing of start or restart of imaging may be a fixed value. However, not necessarily limited to the above-described, the control unit may further perform the detection of fixation detection, and then, may start or restart the continuous imaging at the timing when the fixation stability is detected after the detection of blink. The state of fixation can be detected based on, for example, the observation image of the anterior portion or the fundus.

### <Pre-notice using Fixation Optical System>

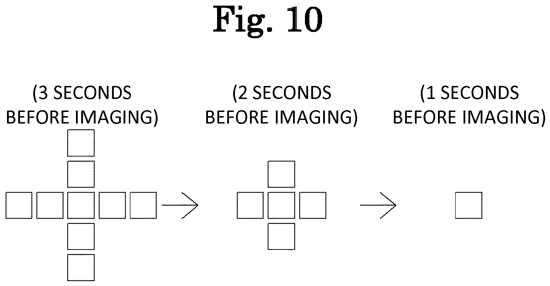

As described above, the control unit may perform the pre-notice for starting or restarting the continuous imaging by controlling the fixation optical system. In this case, the presentation mode of the fixation target may be changed according to the time until start or restart of the continuous imaging. For example, the flashing cycle (time) of the fixation target may be changed according to the time until start or restart of the continuous imaging. In addition, at least one of the shape, size, and position of the fixation target may be changed according to the time until start or restart of the continuous imaging (refer to Fig. 10). The subject can receive the pre-notice from the fixation target without taking notice by the pre-notice using the fixation target.

### <Drive Unit (Alignment Adjustment Unit)>

A drive unit is a mechanism for changing (adjusting) a positional relationship (alignment state) between the subject eye and the imaging optical system. In the present embodiment, the positional relationship between a passing range of the illumination light and the return light and the pupil, eyebrows and eyelashes of the subject eye is changed according to the alignment state. When the observation optical system is used, in the observation optical system, the positional relationship between the imaging optical system and the subject eye is changed in a constant level.

The drive unit may include an actuator that changes the positional relationship between the subject eye and the imaging optical system based on the signal from the control unit. In addition, the drive unit may be a mechanical mechanism that changes the positional relationships between the subject eye and the imaging optical system according to a force given by the examiner.

The drive unit may, for example, displace an imaging unit including the imaging optical system (and the anterior portion observation optical system), may displace a face support unit (for example, a chin rest) that supports a face of the subject, or may be a combination of both.

For example, in a typical method of the alignment adjustment, first, the positional relationship is performed in the vertical and horizontal directions (referred to as the XY direction), and then, the positional relationship in the longitudinal direction (referred to as the working distance direction, the Z direction) is adjusted. In the adjustment in the vertical and horizontal directions, for example, the optical axis of the imaging optical system is adjusted to coincide with the visual axis of the subject eye. In addition, in the adjustment in the longitudinal direction, a distance from the imaging optical system to the subject eye is adjusted so as to become a predetermined working distance. Various alignment indices may be used in the alignment adjustment. In this case, the fundus imaging apparatus in the present embodiment may include an index projection optical system that projects the alignment index onto the subject eye.

### <Ophthalmologic System>

In the embodiment of the fundus imaging apparatus described above, both the continuous imaging of the fundus image and the composition of fundus images (production of the composite image) are performed by the fundus imaging apparatus. However, the present disclosure is not necessarily limited thereto. For example, the continuous imaging and the composition of the image may be separately performed by the fundus imaging apparatus and the ophthalmologic computer other than the fundus imaging apparatus. The fundus imaging apparatus and the ophthalmologic computer may be connected to each other via a network such as a LAN or a WAN.

In the ophthalmologic system, the fundus imaging apparatus includes at least the imaging optical system and the control unit. On the other hand, the ophthalmologic computer has the image processing unit.

The control unit of the fundus imaging apparatus can appropriately perform the action items described subsequent to the "imaging operation" above. On the other hand, the ophthalmologic computer acquires a plurality of fundus images that can be obtained by continuous imaging performed by the fundus imaging apparatus, from the fundus imaging apparatus. In addition, the ophthalmologic computer produces a composite image of a plurality of fundus images using the image processing unit.

### [Application Example]

Hereinafter, one typical application example of the present disclosure will be described with reference to the drawings. First, the overall configuration of a fundus imaging apparatus 1 will be described with reference to Figs. 1 to 3.

### <External Configuration>

As illustrated in Fig. 1, in the present application example, an apparatus main body includes a imaging unit 4, a position alignment mechanism 5, a base 6, and a face support unit 7. In the imaging unit 4, an optical system for imaging a subject eye E is stored. This optical system will be described later with reference to Figs. 2and 3.

The position alignment mechanism 5 ("moving mechanism" in the present application example) is used to align the apparatus with the subject eye E. In the present application example, the position alignment mechanism 5 three-dimensionally moves the imaging unit 4 relative to the base 6. The imaging unit 4 may be movable in each of the Y direction (vertical direction), the X direction (horizontal direction), and the Z direction (longitudinal direction). Unless otherwise noted, in the following description, the position alignment mechanism 5 is assumed to be manually driven based on an operation by a joystick 8 or the like.

The face support unit 7 supports the examinee's face in a state in which the subject eye E is facing the imaging unit 4 as illustrated in Fig. 1. In the present application example, the face support unit 7 is fixed relative to the base 6.

### <SLO Optical System>

In the present application example, the fundus imaging apparatus 1 includes a front imaging optical system 200 (refer to Fig. 2 and Fig. 3) for capturing a front image of the fundus. In the present application example, the front imaging optical system is a scanning laser ophthalmoscope (SLO) optical system. In addition, in the present application example, the front imaging optical system 200 also acts as a fixation optical system (internal fixation optical system). The SLO optical system 200 acquires a front image of the fundus Er by scanning the fundus Er with the laser light and receiving the return light of the laser light from the fundus Er.

In the descriptions below, the fundus imaging apparatus 1 acquires the fundus image by two-dimensionally performs the scanning with the laser light collected on spots on the observation plane based on the operation of a scanning unit (light scanner). However, not necessarily limited thereto, the SLO optical system 200 may be a so-called line scan type optical system. In this case, the observation plane is scanned by a linear light flux. In addition, as in this application example, not a scanning optical system but a non-scanning optical system may be used as the front image imaging optical system.

The SLO optical system 200 includes an emission optical system 10 and a light receiving optical system 20.

First, with reference to Fig. 2, an optical system in a case where an imaging angle of view is a first angle of view will be described. In the present application example, when an attachment optical system 3 (refer to Fig. 3) is not attached, the imaging angle of view is set to be the first angle of view.

The emission optical system 10 includes a scanning unit 16 and an objective optical system 17. The objective optical system 17 in the present application example includes at least one lens. In addition, as illustrated in Fig. 2, the emission optical system 10 further includes a laser light source 11, an aperture mirror 13, a lens 14 (a part of a diopter correction unit 40 in the present application example), and a lens 15.

The laser light source 11 is a light source of the emission optical system 10. In the present application example, the laser light from the laser light source 11 is used as the imaging light. The laser light source 11 in the present application example can emit the light of a plurality of colors simultaneously or selectively. As an example, in the present application example, the laser light source 11 emits the light of total four colors: three colors in the visible range of blue, green, and red, and one color in the infrared range. The light of each color may be able to be emitted simultaneously in any combination. In the present application example, the light of each color emitted from the laser light source 11 is used for imaging the fundus. In some cases, a fundus image captured based on the fundus reflection light is referred to as a reflection image, and a fundus image captured based on fluorescence from a fluorescent substance existing in the fundus Er is referred to as a fluorescent image.

As the reflection image, all or any of an infrared image, a color image, a red free image, and a single color visible image may be captured. In addition, as the fluorescent image, all or any of a fluorescent contrast image and a spontaneous fluorescent image may be captured. The fluorescent contrast image may be an image of the fluorescence of contrast agent intravenously injected into the fundus Er, may be, for example, a fluorescein angiographic (FA) image, or may be an indocyanine green contrast image (ICGA) image. In addition, the spontaneous fluorescent image may be an image by the fluorescence of the fluorescent substance accumulated in the fundus Er, or may be, for example, an image by the fluorescence of lipofuscin.

In the present application example, the laser light from the laser light source 11 passes through the lens 14 and the lens 15 via an opening portion formed in the aperture mirror 13, and then, advances to the scanning unit 16. The laser light reflected by the scanning unit 16 passes through a dichroic mirror 51, passes through the objective optical system 17, and then, is radiated to the fundus Er of the subject eye E. As a result thereof, the laser light excites the fluorescent substance that is reflected and/or scattered from the fundus Er or that is present in the fundus Er, to generate the fluorescence from the fundus. These lights (that is, reflected and/or scattered light, the fluorescence, and the like) are emitted from the pupil as the light from the subject eye (return light) associated with the irradiation of the laser light.

The scanning unit 16 (also referred to as a "light scanner") is a device for scanning the fundus Er with the laser light emitted from laser light source 11. In the description described above, unless otherwise noted, it is assumed that the scanning unit 16 includes two light scanners of which the scanning directions of the laser light are different from each other. That is, a light scanner 16a for main scanning (for example, for scanning in the X direction) and a light scanner 16b for sub-scanning (for example, for scanning in the Y direction) are included. In the following description, it is assumed that the light scanner 16a for main scanning is a resonant scanner and the light scanner 216b for sub-scanning is a galvano mirror. However, another light scanner may be applied to each light scanner 16a and 16b. For example, an acousto-optic device (AOM) or the like that changes the traveling (deflection) direction of the light may be applied to each of the light scanners 16a and 16b in addition to other reflection mirrors (galvano mirrors, polygon mirrors, resonant scanners, MEMS, and the like).

The objective optical system 17 is an objective optical system of the fundus imaging apparatus 1. The objective optical system 17 is used for irradiating the subject eye E with the laser light scanned by the scanning unit 16 and leading the laser light to the fundus Er. For that purpose, the objective optical system 17 forms a turning point P at which the laser light passed through the scanning unit 16 is turned. The turning point P is formed on a reference optical axis L1 of the emission optical system 10 which is a position optically conjugate with the scanning unit 16 with respect to the objective optical system 17. The "conjugate" in the present disclosure, is not necessarily limited to a perfect conjugate relationship, and includes a "substantially conjugate". That is, even in a case where the perfect conjugate position is disposed in a deviated manner within the allowable range in relation to a purpose (for example, observation, analysis, or the like) of using the fundus image, the "conjugate" in that case also included in the "conjugate" in the present disclosure. In the present application example, the objective optical system 17 of the fundus imaging apparatus 1 is realized only by the lens, but not limited thereto, and may be realized by a combination of the lens and mirror.

The laser light passed through the scanning unit 16 is radiated to the fundus Er through the turning point P by passing through the objective optical system 17. Therefore, the laser light passed through the objective optical system 17 is turned around the turning point P in accordance with the operation of the scanning unit 16. As a result, in the present application example, the fundus Er is two-dimensionally scanned with the laser light. The laser light radiated to the fundus Er is collected at a collection position (for example, the surface of the retina).

Next, the light receiving optical system 20 will be described. The light receiving optical system 20 includes one or a plurality of light receiving elements. For example, as illustrated in Fig. 2, the light receiving optical system 20 may include a plurality of light receiving elements 25, 27, and 29. In this case, the light from the fundus Er radiated with the laser light by the emission optical system 10 is received by at least one of the light receiving elements 25, 27, and 29.

As illustrated in Fig. 2, the light receiving optical system 20 in the present application example may share each member disposed from the objective optical system 17 to the aperture mirror 13 with the emission optical system 10. In this case, the light from the fundus Er is guided to the aperture mirror 13 by going back along the optical path of the emission optical system 10. The aperture mirror 13 guides the light from the fundus Er to an independent optical path of the light receiving optical system 20 while removing at least a part of the noise light due to the reflection from a cornea of the subject eye E and the optical system in the apparatus (for example, the lens surface of the objective lens system).

AS the optical path branching member for branching the emission optical system 10 and the light receiving optical system 20, not limited to the aperture mirror 13, but other optical members (for example, a half mirror or the like) may be used.

The light receiving optical system 20 in the present application example includes a lens 21, a pinhole plate 23, and a light separation unit (light separation unit) 23 in a reflection optical path of the aperture mirror 13.

The pinhole plate 23 is disposed in the fundus conjugate plane, and functions as a confocal stop in the fundus imaging apparatus 1. That is, when the diopter is properly corrected by the diopter correction unit 40, the light from the fundus Er which passed through the lens 21 is focused at the aperture of the pinhole plate 23. The pinhole plate 23 removes the light from the positions other than the focal point (or focal plane) of the fundus Er, and the remainder (light from the focal point) is guided to at least one of the light receiving elements 25, 27, and 29.

A light separation unit 23 separates the light that comes from the fundus Er. In the present application example, the light from the fundus Er is wavelength-selectively separated by the light separation unit 23. In addition, the light separation unit 23 may also be used as a light branch unit for branching the optical path of the light receiving optical system 20. For example, as illustrated in Fig. 2, the light separation unit 23 may include two dichroic mirrors (dichroic filters) 31 and 32 of which the light separation characteristics (wavelength separation characteristics) are different from each other. The optical path of the light receiving optical system 20 branches into three by the two dichroic mirrors 31 and 32. In addition, at the end of each branch optical path, one of the light receiving elements 25, 27, and 29 is disposed, respectively.

For example, the light separation unit 23 separates the wavelengths of light from the fundus Er, and causes the three light receiving elements 25, 27, and 29 to receive the light of which the wavelength ranges are different from each other. For example, the light receiving elements 25, 27, and 29 may be caused to receive the light of three colors of blue, green and red one by one. In this case, a color image may be acquired from the result of light reception by each of the light receiving elements 25, 27, and 29.

In addition, the light separation unit 23 may cause the light receiving elements different from each other to receive the light of the fundus spontaneous fluorescence and the infrared light which is the fundus reflection light of the observation light. In this way, it may be possible to capture an infrared image simultaneously with the fluorescent image. In the present application example, the blue light emitted from the laser light source 11 is used as excitation light for the spontaneous fluorescence.

The control unit 70 forms a fundus image based on the light reception signals output from, for example, the light receiving elements 25, 27, and 29. Specifically, the control unit 70 forms a fundus image in synchronization with the light scanning by the scanning unit 16. For example, the control unit 70 forms a fundus image of at least one frame (in other words, one sheet) (for each light receiving element) every time the light scanner 16b for sub-scanning reciprocates n times (n is an integer of equal to or greater than 1). In the following, unless otherwise noted, it is assumed that one frame of the fundus image based on one reciprocation of light scanner 16b for sub-scanning is formed for convenience. In the present application example, since three light receiving elements 25, 27, and 29 are provided, the control unit 70 produces maximum three types of images based on the signals from the respective light receiving elements 25, 27, and 29 every time the light scanner 16b for sub-scanning reciprocates.

The control unit 70 may cause a monitor 80 to display a plurality of frames of the fundus images which are sequentially formed based on the above-described operations of the apparatus, as an observation image in time series. The observation image is a dynamic image consisting of a fundus image acquired substantially in real time. In addition, the control unit 70 takes (captures) a part of a plurality of sequentially formed fundus images as a captured image (capture image). At this time, the captured image is stored in the storage medium. The storage medium in which the captured image is stored may be a non-volatile storage medium (for example, hard disk, flash memory, or the like). In the present application example, for example, after the output of a trigger signal (for example, a releasing operation signal or the like), a fundus image formed at a predetermined timing (or period) is captured.

Next, an alignment optical system 50 will be described. As an example, the alignment optical system 50 in the present application example includes a dichroic mirror 51, an alignment light source 52, and a light receiving element 55. In addition, in the present application example, as an example, the alignment optical system 50 may include a lens 53 as an optical element for magnification correction of the alignment index.

The alignment light emitted from the alignment light source 52 is reflected by the dichroic mirror 51 and radiated toward the subject eye E through the objective optical system. The alignment light reflected by the cornea of the subject eye E passes through the objective optical system and is reflected by the dichroic mirror 51, and then, is received by the light receiving element 55 through the lens 53. Based on the result of light reception by the light receiving element 55, the alignment state of the subject eye E with respect to the objective optical system is detected. The signal output from the light receiving element 55 is input to the control unit 70 (refer to Fig. 4). The control unit 70 may produce an image indicating the alignment state of the subject eye E (hereinafter also referred to as "alignment image") based on the input signal. The alignment image may be, for example, an image that includes the alignment index image indicating the alignment light reflected by the cornea of the subject eye E.

Here, the lens 53 is movable along the optical axis, the focal length of the alignment optical system can be changed and the magnification of the alignment index image can be switched using the lens 53. The drive unit 53a for moving the lens 53 may be provided in the fundus imaging apparatus 1. The lens 53 is displaced according to the attachment / detachment (insertion / removal) of the attachment optical system 3 (that is, according to switching of the angle of view).

In the present application example, the fundus imaging apparatus 1 does not include an anterior portion observation optical system. However, of course, the anterior portion observation optical system may be provided in the fundus imaging apparatus 1. In this case, a part of the alignment optical system 50 may be the anterior portion observation system.

Next, with reference to Fig. 3, an optical configuration in a case where an imaging angle of view is a second angle of view having a wider angle will be described. In the present application example, an objective optical system 18 for imaging in a second angle of view is configured by disposing the attachment optical system 3 between the objective optical system 17 and the subject eye E. The attachment optical system 3 in the present application example includes at least one lens. As illustrated in Fig. 3, the attachment optical system 3 may include a plurality of lenses.

### <Attachment Optical System>

By attaching and detaching (inserting and removing) a lens attachment including the attachment optical system 3 to and from a housing surface of the imaging unit 4, the insertion and removal of the attachment optical system 3 is performed between the objective lens 17 on the main body side of the apparatus and the subject eye E.

At least the lens 332 bends the measurement light that passed a first turning point P1 toward the optical axis L1, a second turning point P2 is formed at a position conjugate to the light scanner 16 with respect to the attachment optical system 3 and the objective optical system 17. That is, the attachment optical system 3 is an optical system that relays the turning point P1 to the turning point P2.

In the present application example, a turning amount of the measurement light at the second turning point P2 is larger than a turning amount at the first turning point P1. In the present application example, the angle of view of the apparatus is defined by the turning amount. In the present application example, the angle of view (turning amount) is approximately 60° in the retracted state (first mode), and the angle of view (turning amount) is approximately 100° in the inserted state (second mode).

### <Control System>

As illustrated in Fig. 4, the fundus imaging apparatus 1 further includes an arithmetic controller (arithmetic control unit) 70. In addition, the fundus imaging apparatus 1 may be provided with a memory 71, a monitor 80, an operation unit 75, and the like may be provided. In addition, the arithmetic controller (hereinafter, control unit) 70 is connected to the laser light source 11, the front imaging optical system 200 and the like. The operation unit 75 may be a touch panel, a mouse, a keyboard or the like. The operation unit 75 may be a device separate from the fundus imaging apparatus 1. The control unit 70 may control each part based on the operation signal output from the operation unit 75. For example, any one of an operation for selecting an imaging mode, an operation for release, and the like may be input to the operation unit 75. In addition, in the present application example, various types of image processing are performed by the control unit 70.

### <Operation>

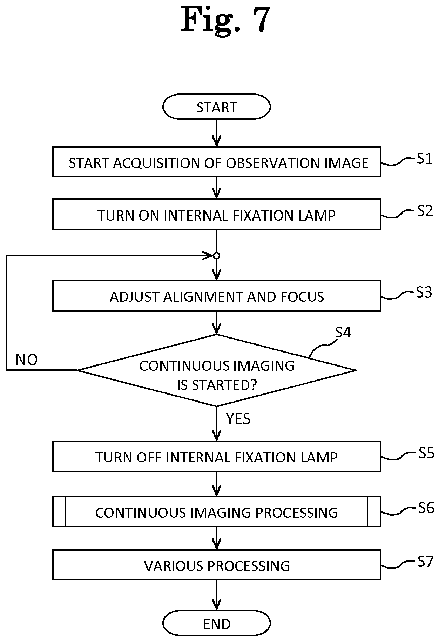

Next, the operation by the fundus imaging apparatus 1 will be described with reference to the flowchart in Fig. 7. For convenience of explanation, here, it is assumed that the imaging method is selected in advance. In detail, it is assumed that an imaging method for performing the continuous imaging using the visible light as the illumination light is selected. In addition, it is assumed that the face of the examinee is supported by the face support unit 7 prior to the operation described below.

Furthermore, in the description below, for the convenience, it is assumed that the composite image acquired based on the continuous imaging is the addition average image.

First, the control unit 70 starts the acquisition of the observation image (S1), and turns on the internal fixation target (S2). The control unit 70 irradiates the subject eye with the infrared light as the observation light and scans the fundus with the infrared light to obtain a fundus observation image. In addition, the control unit 70 controls, for example, the imaging optical system to temporarily turn on the visible light at the timing when a predetermined presentation position is scanned with the laser. In this way, an internal fixation target is formed and presented to the subject eye.

Next, the alignment state and the focus state are adjusted using the observation image (S3). In the present application example, the adjustment is performed until a predetermined imaging trigger is issued (No in S4). After the alignment state and the focus state are appropriately adjusted, the continuous imaging is started based on a predetermined imaging trigger (Yes in S4). In the present application example, after the internal fixation target is turned off by the control unit 70 (S5), the continuous imaging processing (S5) is performed. In the continuous imaging processing, an operation at the time of continuous imaging in the present application example is defined.

### <Continuous Imaging Processing>

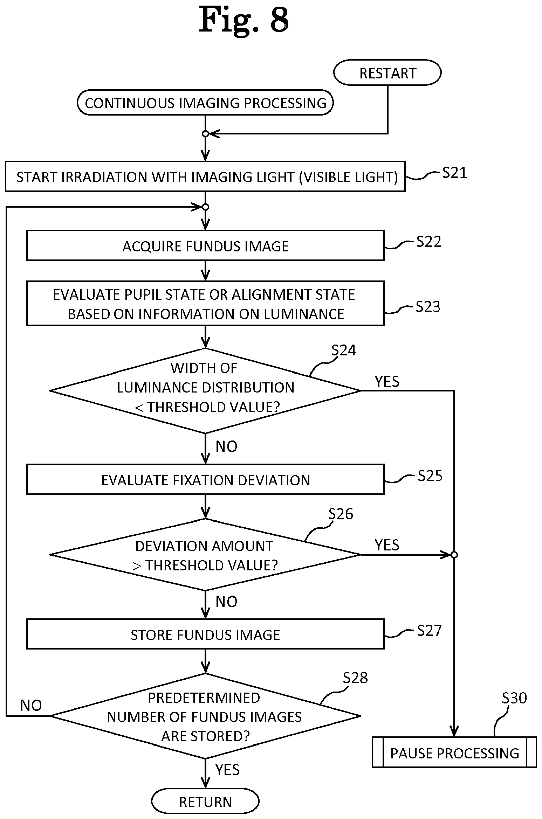

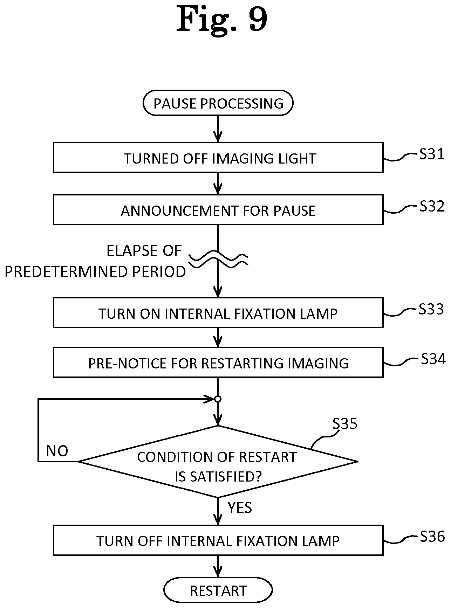

Here, the continuous imaging processing (S5) will be described in detail with reference to Fig. 8 and Fig. 9. The continuous imaging processing in the present application example is processing for acquiring and storing a predetermined number of fundus images.

First, the control unit 70 starts the emission of the visible light (the illumination light) to the fundus of the subject eye E (S21).

Then, the control unit 70 drives and controls the scanning unit 16 and acquires (captures) one fundus image based on the return light of the illumination light (S22). In the present application example, the imaging is repeated through the processing in S23 to S28.

While the repeated imaging is performed, the state of the subject eye at the time of capturing the fundus image is evaluated by the control unit 70 through the processing in S23 to S26. In the present application example, the stats at the time of capturing the fundus image is evaluated based on the fundus image captured immediately before.

As an example, in the present application example, the information on the luminance of the fundus image is acquired as the evaluation information on the miosis or the misalignment. Then, the miosis or alignment deviation is evaluated based on the information on the luminance (S23), and it is determined whether the miosis or alignment deviation equal to or greater than a reference has occurred or not (S24). In particular, the width (or variance or deviation) of the histogram (luminance distribution) in the raw data of the fundus image is used to evaluate the miosis or alignment deviation. Here, as the width of the histogram is narrower, the miosis or alignment deviation is considered to be larger. The width is compared with a threshold value, and if the width is equal to or wider than the threshold value, the process proceeds to S25 (if No in S24 → S25), and if less than the threshold value, the process proceeds to pause processing (Yes in S24 → S30).

Next, in the present application example, a deviation amount between the fundus image captured immediately before and a template fundus image is acquired (S25) as the evaluation information on the fixation deviation. The template fundus image may be, for example, a fundus image captured first after the continuous imaging is started. The deviation amount may be information indicated by the number of pixels, an angle or the like. Next, the control unit 70 determines whether or not the deviation amount exceeds a threshold value (S26). If the deviation amount exceeds the threshold value (Yes in S26), the pause processing (S30) is performed and the continuous imaging is paused (temporarily stopped). On the other hand, if the deviation amount is equal to or smaller than the threshold value (No in S26), the process proceeds to S27.

In the processing in S27, the fundus image captured immediately before is stored in the memory. That is, in the present application example, the fundus image captured when the miosis or alignment deviation is within the allowable range (No in S24) and when the fixation deviation is within the allowable range (No in S26), is stored in the memory.

After the storage, the control unit 70 determines whether or not the number of fundus images stored from the start of continuous imaging reaches a predetermined number (S28). If the number is not enough, the process returns to S22, and the processing is repeated. In addition, when the predetermined number of images are stored, the continuous imaging processing is ended, and the processing in S7 (various processing) are performed.

In the present application example, when process proceeds to the pause processing (S30), the fundus image captured immediately before is not stored in the memory 71. In this way, it is suppressed that the unwanted fundus image is mixed into a plurality of fundus images that become the basis of the addition image.

### <Pause Processing>

In the present application example, while waiting for the recovery of the state of the subject eye E, the pause processing (S30) is performed.

First, the illumination light is turned off (S31). In this way, the recovery from miosis is expected. In addition, in the present application example, the control unit 70 outputs an announcement for pause from a speaker or the like (S32). This can release the examinee's strain and prompt the recovery from the miosis and fatigue.

In the present application example, as an example, the period of pause is set to a substantially constant period. After the predetermined period elapsed, the control unit 70 may turn on the internal fixation lamp (S33) and perform the pre-notice for restarting the imaging (S34). The appropriate fixation allows the imaging of the same range as before the pause. Also, in the present application example, the restart of imaging is pre-noticed by the sound from the speaker or the like. For example, "Now, imaging is restarted. Please put your face on the chin table." or the like may be output. In the present application example, a sensor (not illustrated) for detecting whether a face is on the chin table may be provided in the face support unit 7. When it is detected that the face is on the chin table, the control unit 70 may further control the SLO optical system 200 to perform the pre-notice using the fixation target.

As illustrated in Fig. 10, in the present application example, the presentation mode of the fixation target changes according to the time until imaging. Specifically, at least the shape of the fixation target changes stepwise. For example, as illustrated in Fig. 10, it changes at every second in an order from 3 seconds before restarting the imaging. In the present application example, the shape of the fixation target converges with respect to the targeted fixation position. As a result, it is possible to suppress the blurring of the fixation by following the changing fixation target with the line of sight. Furthermore, in addition to the change of the fixation target, the speaker may output sound such as "3, 2, 1". As a result, the examinee can easily grasp the timing of restarting the imaging, and it is possible to prompt the appropriate fixation and eye opening in preparation for imaging.

In In the present application example, the lapse of a fixed time triggers the imaging restart. If the imaging is restarted (Yes in S35), the internal fixation light is turned off (S36), and the process returns to the continuous imaging processing. In this case, the process proceeds to S21 and the processing is restarted.

### <Production of Addition Image>

In various processing (S7) performed after the completion of the continuous imaging processing in the present application example, for example, the addition average image may be produced using a plurality of fundus images stored in the memory 71 in a series of continuous imaging processing. The produced addition average image may be stored in the memory 71 or may be displayed on the monitor 80. In the present application example, in any of the plurality of fundus images that become the basis of the addition average image, the states of the subject eye at the time of imaging are within the allowable range, it is possible to enjoy a good image quality of the addition average image.

### <Modification Example>

As above, the present disclosure has been described based on the embodiment and application examples. However, the present disclosure is not limited to the above embodiment and application examples, and various modifications are allowed based on a so-called ordinary knowledge of the person skilled in the art.

For example, if the fundus imaging apparatus includes two imaging optical systems, the two imaging optical systems may be capable of imaging simultaneously. The evaluation information in the continuous imaging by one imaging optical system may be obtained from an image of the subject eye captured by the other imaging optical system during the continuous imaging. Here, as an example, the evaluation information in the continuous imaging of a front imaging optics that captures a front image of the fundus may be acquired based on a tomographic image of the fundus captured by the OCT optical system. For the optical system of such an apparatus, refer to, for example, "JP-A-2016-104105" by the present applicant.

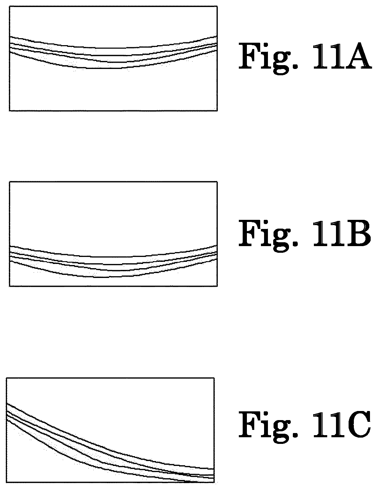

In this case, two imaging optical systems may perform the continuous imaging in parallel. An example of a tomographic image obtained when the state of the subject eye is correct is illustrated in Fig. 11A. In addition, an example of a tomographic image when the alignment state changes in the longitudinal direction compared to Fig. 11A, is illustrated in Fig. 11B, and an example of a tomographic image when the alignment state changes in the vertical direction is illustrated in Fig. 11C.

As illustrated in Fig. 11A and Fig. 11C, when the subject eye moves away from the apparatus, the position of the fundus tissue in the image is displaced in the depth direction. Therefore, the change in the alignment state can be evaluated based on the displacement amount in the depth direction between the tomographic images.

In addition, as illustrated in Fig. 11A and Fig. 11B, when the subject eye moves in the vertical and horizontal directions, the incidence angle of the measurement light with respect to the fundus changes and thus, the image is tilted. Therefore, based on the displacement amount in the rotational direction between the tomographic images, it is possible to evaluate the change in the alignment state relating to the vertical and horizontal directions.

## Claims

1. A fundus imaging apparatus (1) comprising:
an imaging optical system (200) that irradiates a fundus (Er) of a subject eye (E) with illumination light and captures a fundus image based on return light of the illumination light from the fundus (Er);
a control unit (70) that controls the imaging optical system (200) to perform continuous imaging of the fundus (Er) based on an imaging trigger,
wherein the control unit (70) sequentially acquires evaluation information used as an evaluation index of a state of the subject eye (E) during the continuous imaging, determines whether or not to stop the continuous imaging based on the evaluation information, and continues or stops the continuous imaging according to a result of the determination; and
an image processing unit (70) that produces a composite image composed of a plurality of fundus images obtained by the continuous imaging.

2. The fundus imaging apparatus (1) according to claim 1,
wherein the evaluation information relates to at least one of a pupil state, an alignment state, and a fixation state of the subject eye (E).

3. The fundus imaging apparatus (1) according to claim 1,
wherein the control unit (70) acquires the evaluation information based on at least a fundus image acquired during the continuous imaging.

4. The fundus imaging apparatus (1) according to claim 1 or 2, further comprising:
an anterior portion observation optical system for observing an anterior portion of the subject eye (E),
wherein the control unit (70) acquires the evaluation information based on at least an anterior portion observation image obtained by an observation through the anterior portion observation optical system during the continuous imaging.

5. The fundus imaging apparatus (1) according to any one of claims 1 to 3, further comprising:
a fundus observation optical system for observing the fundus (Er),
wherein the control unit (70) acquires the evaluation information based on at least a fundus observation image obtained by an observation through the fundus observation optical system during the continuous imaging.

6. The fundus imaging apparatus (1) according to any one of claims 1 to 3, further comprising:
a second imaging optical system that captures a fundus image different from the fundus image at the same time of imaging by the imaging optical system (200),
wherein the control unit (70) acquires the evaluation information based on at least a fundus image captured by the second imaging optical system during the continuous imaging.

7. The fundus imaging apparatus (1) according to any one of claims 1 to 6,
wherein the control unit (70) restarts the continuous imaging after stopping the continuous imaging based on the determination.

8. The fundus imaging apparatus (1) according to claim 7,
wherein the control unit (70) sequentially acquires the evaluation information while the continuous imaging is stopped, and determines whether or not to restart the continuous imaging based on the evaluation information acquired during the stoppage.

9. The fundus imaging apparatus (1) according to claim 7 or 8, further comprising:
a fixation optical system (200) that shares a part of optical paths (L1, L2) with the imaging optical system (200) and projects an internal fixation target onto the subject eye (E),
wherein the control unit (70) controls the fixation optical system (200) to present the internal fixation target while the continuous imaging is stopped.

10. The fundus imaging apparatus (1) according to any one of claims 1 to 9, further comprising:
an output unit (80) that outputs information to an examiner or an examinee,
wherein when the control unit (70) starts or restarts the continuous imaging, the control unit (70) performs a pre-notice for starting or restarting the continuous imaging via the output unit (80).

11. The fundus imaging apparatus (1) according to claim 10,
wherein the control unit (70) causes the output unit (80) to output the pre-notice including information for prompting the subject eye (E) to blink, and
when the control unit (70) detects a blink after the pre-notice is output, the control unit (70) starts or restarts the continuous imaging.

12. The fundus imaging apparatus (1) according to claim 11,
wherein the control unit (70) starts or restarts the continuous imaging after the blink is detected and a fixation is stabilized.

13. The fundus imaging apparatus (1) according to any one of claims 1 to 12,
wherein the illumination light is visible light.

14. A method of producing a composite image composed of a plurality of fundus images by the fundus imaging apparatus as disclosed in the claims 1-13 (1) including an imaging optical system (200) that irradiates a fundus (Er) of a subject eye (E) with illumination light and captures a fundus image based on return light of the illumination light from the fundus (Er), the method comprising:
a continuous imaging step of controlling the imaging optical system (200) by a control unit (70) of the fundus imaging apparatus (1) to perform continuous imaging of the fundus (Er) based on an imaging trigger;
an evaluation information acquisition step of acquiring evaluation information used as an evaluation index of a state of the subject eye (E) during the continuous imaging by the control unit (70);
a determination step of determining whether or not to stop the continuous imaging based on the evaluation information by the control unit (70);
a stop step of stopping the continuous imaging when it is determined to stop the continuous imaging; and
an image processing step of producing a composite image composed of a plurality of fundus images obtained by the continuous imaging by an image processing processor of the fundus imaging apparatus (1).

## Patentansprüche

1. Fundusabbildungsvorrichtung (1), umfassend:
ein optisches Abbildungssystem (200), das einen Fundus (Er) eines Gegenstandsauges (E) mit Beleuchtungslicht bestrahlt und ein Fundusbild basierend auf dem vom Fundus (Er) zurückkehrenden Licht des Beleuchtungslichtes aufnimmt,
eine Steuereinheit (70), die das optische Abbildungssystem (200) steuert, um eine kontinuierliche Abbildung des Fundus (Er) basierend auf einem Abbildungsauslöser durchzuführen,
wobei die Steuereinheit (70) sequentiell Evaluierungsinformationen erfasst, die als ein Evaluierungsindex eines Zustands des Gegenstandsauges (E) bei der kontinuierlichen Abbildung verwendet werden, bestimmt, ob die kontinuierliche Abbildung basierend auf den Evaluierungsinformationen zu stoppen ist oder nicht, und die kontinuierliche Abbildung gemäß einem Ergebnis der Bestimmung fortsetzt oder stoppt, und
eine Bildverarbeitungseinheit (70), die ein zusammengesetztes Bild erzeugt, das eine Mehrzahl von Fundusbildern aufweist, die durch die kontinuierliche Abbildung aufgenommen wurden.

2. Fundusabbildungsvorrichtung (1) nach Anspruch 1,
wobei sich die Evaluierungsinformationen auf mindestens einen der folgenden Zustände beziehen: einen Pupillenzustand, einen Ausrichtungszustand und einen Fixierungszustand des Gegenstandauges (E).

3. Fundusabbildungsvorrichtung (1) nach Anspruch 1,
wobei die Steuereinheit (70) die Evaluierungsinformationen basierend auf mindestens einem Fundusbild erfasst, das bei der kontinuierlichen Abbildung aufgenommen wurde.

4. Fundusabbildungsvorrichtung (1) nach Anspruch 1 oder 2, ferner umfassend:
ein optisches Vorderabschnitt-Beobachtungssystem zum Beobachten eines Vorderabschnitts des Gegenstandsauges (E),
wobei die Steuereinheit (70) die Evaluierungsinformationen basierend auf mindestens einem Vorderabschnitt-Beobachtungsbild erfasst, das durch eine Beobachtung durch das optische Vorderabschnitt-Beobachtungssystem bei der kontinuierlichen Abbildung aufgenommen wurde.

5. Fundusabbildungsvorrichtung (1) nach einem der Ansprüche 1 bis 3, ferner umfassend:
ein optisches Fundusbeobachtungssystem zur Beobachtung des Fundus (Er),
wobei die Steuereinheit (70) die Evaluierungsinformationen basierend auf mindestens einem Fundusbeobachtungsbild erfasst, das durch eine Beobachtung durch das optische Fundusbeobachtungssystem bei der kontinuierlichen Abbildung aufgenommen wurde.

6. Fundusabbildungsvorrichtung (1) nach einem der Ansprüche 1 bis 3, ferner umfassend:
ein zweites optisches Abbildungssystem, das ein Fundusbild aufnimmt, das sich von dem Fundusbild zum gleichen Zeitpunkt der Abbildung durch das optische Abbildungssystem (200) unterscheidet,
wobei die Steuereinheit (70) die Evaluierungsinformationen basierend auf mindestens einem Fundusbild erfasst, das von dem zweiten optischen Abbildungssystem bei der kontinuierlichen Abbildung aufgenommen wurde.

7. Fundusabbildungsvorrichtung (1) nach einem der Ansprüche 1 bis 6,
wobei die Steuereinheit (70) die kontinuierliche Abbildung neu startet, nachdem die kontinuierliche Abbildung basierend auf der Bestimmung gestoppt wurde.

8. Fundusabbildungsvorrichtung (1) nach Anspruch 7,
wobei die Steuereinheit (70) sequentiell die Evaluierungsinformationen erfasst, wenn die kontinuierliche Abbildung gestoppt ist, und basierend auf den bei der Unterbrechung erfassten Evaluierungsinformationen bestimmt, ob die kontinuierliche Abbildung neu zu starten ist oder nicht.

9. Fundusabbildungsvorrichtung (1) nach Anspruch 7 oder 8, ferner umfassend:
ein optisches Fixierungssystem (200), das einen Abschnitt der optischen Pfade (L1, L2) mit dem optischen Abbildungssystem (200) teilt und ein internes Fixierungsziel auf das Gegenstandsauge (E) projiziert,
wobei die Steuereinheit (70) das optische Fixierungssystem (200) steuert, um das interne Fixierungsziel zu präsentieren, wenn die kontinuierliche Abbildung gestoppt ist.

10. Fundusabbildungsvorrichtung (1) nach einem der Ansprüche 1 bis 9, ferner umfassend:
eine Ausgabeeinheit (80), die Informationen an einen Prüfer oder einen Prüfling ausgibt,
wobei, wenn die Steuereinheit (70) die kontinuierliche Abbildung startet oder neu startet, die Steuereinheit (70) eine Vorankündigung für das Starten oder Neustarten der kontinuierlichen Abbildung über die Ausgabeeinheit (80) durchführt.

11. Fundusabbildungsvorrichtung (1) nach Anspruch 10,
wobei die Steuereinheit (70) die Ausgabeeinheit (80) veranlasst, die Vorankündigung auszugeben, die Informationen zur Aufforderung des Gegenstandsauges (E) zum Blinzeln aufweist, und
wenn die Steuereinheit (70) nach der Ausgabe der Vorankündigung ein Blinzeln detektiert, startet oder neu startet die Steuereinheit (70) die kontinuierliche Abbildung.

12. Fundusabbildungsvorrichtung (1) nach Anspruch 11,
wobei die Steuereinheit (70) die kontinuierliche Abbildung startet oder neu startet, nachdem das Blinzeln detektiert wurde und eine Fixierung stabilisiert wurde.

13. Fundusabbildungsvorrichtung (1) nach einem der Ansprüche 1 bis 12,
wobei das Beleuchtungslicht ein sichtbares Licht ist.

14. Verfahren zum Erzeugen eines zusammengesetzten Bildes, das eine Mehrzahl von Fundusbildern aufweist, durch die Fundusabbildungsvorrichtung (1) gemäß den Ansprüchen 1 bis 13, umfassend ein optisches Abbildungssystem (200), das einen Fundus (Er) eines Gegenstandsauges (E) mit Beleuchtungslicht bestrahlt und ein Fundusbild basierend auf dem vom Fundus (Er) zurückkehrenden Licht des Beleuchtungslichts aufnimmt, wobei das Verfahren aufweist:
einen kontinuierlichen-Abbildung-Schritt zum Steuern des optischen Abbildungssystems (200) durch eine Steuereinheit (70) der Fundusabbildungsvorrichtung (1), um eine kontinuierliche Abbildung des Fundus (Er) basierend auf einem Abbildungsauslöser durchzuführen,
einen Evaluierungsinformation-Erfassungsschritt zum Erfassen von Evaluierungsinformationen, die als ein Evaluierungsindex eines Zustands des Gegenstandsauges (E) bei der kontinuierlichen Abbildung durch die Steuereinheit (70) verwendet werden,
einen Bestimmungsschritt zum Bestimmen, ob die kontinuierliche Abbildung basierend auf den Evaluierungsinformationen durch die Steuereinheit (70) zu stoppen ist oder nicht,
einen Stoppschritt zum Stoppen der kontinuierlichen Abbildung, wenn bestimmt wird, die kontinuierliche Abbildung zu stoppen, und
einen Bildverarbeitungsschritt zum Erzeugen eines zusammengesetzten Bildes, das eine Mehrzahl von Fundusbildern aufweist, die durch die kontinuierliche Abbildung durch einen Bildverarbeitungsprozessor der Fundusabbildungsvorrichtung (1) aufgenommen wurden.

## Revendications

1. Appareil d'imagerie de fond d'œil (1) comprenant :
un système optique d'imagerie (200) qui irradie un fond d'œil (Er) d'un œil d'un sujet (E) avec une lumière d'éclairage et capture une image de fond d'œil sur la base d'une lumière de retour de la lumière d'éclairage provenant du fond d'œil (Er) ;
une unité de commande (70) qui commande le système optique d'imagerie (200) pour effectuer une imagerie continue du fond d'œil (Er) sur la base d'un déclencheur d'imagerie,
dans lequel l'unité de commande (70) acquiert séquentiellement des informations d'évaluation utilisées comme indice d'évaluation d'un état de l'œil du sujet (E) pendant l'imagerie continue, détermine s'il faut arrêter l'imagerie continue ou non sur la base des informations d'évaluation, et continue ou arrête l'imagerie continue selon un résultat de la détermination ; et
une unité de traitement d'image (70) qui produit une image composite composée d'une pluralité d'images de fond d'œil obtenues par l'imagerie continue.

2. Appareil d'imagerie de fond d'œil (1) selon la revendication 1,
dans lequel les informations d'évaluation concernent au moins l'un d'un état de pupille, d'un état d'alignement et d'un état de fixation de l'œil du sujet (E).

3. Appareil d'imagerie de fond d'œil (1) selon la revendication 1,
dans lequel l'unité de commande (70) acquiert les informations d'évaluation sur la base d'au moins une image de fond d'œil acquise pendant l'imagerie continue.

4. Appareil d'imagerie de fond d'œil (1) selon la revendication 1 ou 2, comprenant en outre :
un système optique d'observation de partie antérieure pour observer une partie antérieure de l'œil du sujet (E),
dans lequel l'unité de commande (70) acquiert les informations d'évaluation sur la base d'au moins une image d'observation de partie antérieure obtenue par une observation à travers le système optique d'observation de partie antérieure pendant l'imagerie continue.

5. Appareil d'imagerie de fond d'œil (1) selon l'une quelconque des revendications 1 à 3, comprenant en outre :
un système optique d'observation de fond d'œil pour observer le fond d'œil (Er),
dans lequel l'unité de commande (70) acquiert les informations d'évaluation sur la base d'au moins une image d'observation de fond d'œil obtenue par une observation à travers le système optique d'observation de fond d'œil pendant l'imagerie continue.

6. Appareil d'imagerie de fond d'œil (1) selon l'une quelconque des revendications 1 à 3, comprenant en outre :
un deuxième système optique d'imagerie qui capture une image de fond d'œil différente de l'image de fond d'œil obtenue au même moment que l'imagerie par le système optique d'imagerie (200),
dans lequel l'unité de commande (70) acquiert les informations d'évaluation sur la base d'au moins une image de fond d'œil capturée par le deuxième système optique d'imagerie pendant l'imagerie continue.

7. Appareil d'imagerie de fond d'œil (1) selon l'une quelconque des revendications 1 à 6,
dans lequel l'unité de commande (70) reprend l'imagerie continue après l'arrêt de l'imagerie continue sur la base de la détermination.

8. Appareil d'imagerie de fond d'œil (1) selon la revendication 7,
dans lequel l'unité de commande (70) acquiert séquentiellement les informations d'évaluation pendant que l'imagerie continue est arrêtée, et détermine s'il faut reprendre l'imagerie continue ou non sur la base des informations d'évaluation acquises pendant l'arrêt.

9. Appareil d'imagerie de fond d'œil (1) selon la revendication 7 ou 8, comprenant en outre :
un système optique de fixation (200) qui partage une partie de trajets optiques (L1, L2) avec le système optique d'imagerie (200) et projette une cible de fixation interne sur l'œil du sujet (E),
dans lequel l'unité de commande (70) commande le système optique de fixation (200) pour présenter la cible de fixation interne pendant que l'imagerie continue est arrêtée.

10. Appareil d'imagerie de fond d'œil (1) selon l'une quelconque des revendications 1 à 9, comprenant en outre :
une unité de sortie (80) qui délivre en sortie des informations à un examinateur ou à une personne examinée,
dans lequel lorsque l'unité de commande (70) commence ou reprend l'imagerie continue, l'unité de commande (70) effectue une pré-notification pour commencer ou reprendre l'imagerie continue via l'unité de sortie (80).

11. Appareil d'imagerie de fond d'œil (1) selon la revendication 10,
dans lequel l'unité de commande (70) amène l'unité de sortie (80) à délivrer en sortie la pré-notification comprenant des informations pour inciter l'œil du sujet (E) à cligner, et
lorsque l'unité de commande (70) détecte un clignement après que la pré-notification est délivrée en sortie, l'unité de commande (70) commence ou reprend l'imagerie continue.

12. Appareil d'imagerie de fond d'œil (1) selon la revendication 11,
dans lequel l'unité de commande (70) commence ou reprend l'imagerie continue après que le clignement est détecté et qu'une fixation est stabilisée.

13. Appareil d'imagerie de fond d'œil (1) selon l'une quelconque des revendications 1 à 12, dans lequel la lumière d'éclairage est une lumière visible.

14. Procédé de production d'une image composite composée d'une pluralité d'images de fond d'œil par l'appareil d'imagerie de fond d'œil tel que divulgué dans les revendications 1 à 13
(1) comprenant un système optique d'imagerie (200) qui irradie un fond d'œil (Er) d'un œil d'un sujet (E) avec une lumière d'éclairage et capture une image de fond d'œil sur la base de la lumière de retour de la lumière d'éclairage provenant du fond d'œil (Er), le procédé comprenant :
une étape d'imagerie continue consistant à commander le système optique d'imagerie (200) par une unité de commande (70) de l'appareil d'imagerie de fond d'œil (1) pour effectuer une imagerie continue du fond d'œil (Er) sur la base d'un déclencheur d'imagerie ;
une étape d'acquisition d'informations d'évaluation consistant à acquérir des informations d'évaluation utilisées comme indice d'évaluation d'un état de l'œil du sujet (E) pendant l'imagerie continue par l'unité de commande (70) ;
une étape de détermination consistant à déterminer s'il faut arrêter l'imagerie continue ou non sur la base des informations d'évaluation par l'unité de commande (70) ;
une étape d'arrêt consistant à arrêter l'imagerie continue lorsqu'il est déterminé qu'il faut arrêter l'imagerie continue ; et
une étape de traitement d'image consistant à produire une image composite composée d'une pluralité d'images de fond d'œil obtenues par l'imagerie continue par un processeur de traitement d'image de l'appareil d'imagerie de fond d'œil (1).
